# EUROPEAN PATENT APPLICATION

(11) **EP 4 699 548 A1**
(43) Date of publication of application: **25.02.2026**
(21) Application number: 24792782.5
(22) Date of filing: 10.01.2024
(51) Int. Cl.: A61B 17/06, A61B 17/00

(54) **ANTIBACTERIAL SUTURE PACKAGING KIT**

(30) Priority: 20.04.2023 KR 20230052141
(71) Applicant: Oh, Jeesoo, Cheongju-si, Chungcheongbuk-do 28162 (KR)
(72) Inventor: Oh, Jeesoo, Cheongju-si, Chungcheongbuk-do 28162 (KR)
(74) Representative: ZBM Patents - Zea, Barlocci & Markvardsen
(86) International application number: PCT/KR2024/000504
(87) International publication number: WO 2024/219594

(57) **Abstract**

An antimicrobial suture package kit of the present disclosure includes a first accommodation unit 140 which has an internal space sealed by a bonded portion 110 formed by welding two synthetic resin sheets; a suture kit 130 accommodated in the first accommodation unit 140; a second accommodation unit 150 which is located to be adjacent to the first accommodation unit 140 and has an internal space sealed by the bonded portion 110; a liquid antimicrobial material 170 accommodated in the second accommodation unit 150; a partition 300 which partially partitions the first accommodation unit 140 and the second accommodation unit 150 so as to ensure a movement path of the antimicrobial material 170; and an antimicrobial capsule 190 which is accommodated in the second accommodation unit 150 and is filled with the antimicrobial material 170 to be swelled by the antimicrobial material 170, and the antimicrobial material 170 is filled in the antimicrobial capsule 190 to be accommodated separately from the suture kit 130 and passes through a movement path formed by the partition 300 when the second accommodation unit 150 is pressed to rupture the antimicrobial capsule 190, and then flows into the suture kit 130, to be absorbed by the suture 120.

## Description

### Technical Field

The present disclosure relates to a suture packaging kit, and more particularly, to an antimicrobial suture packaging kit.

### Background Art

In general, a suture used as a surgical suture material is a thread employed for suturing a damaged portion of a tissue due to surgical trauma. Such a suture is mainly used to fix skin, blood vessels, tissues, and organs, or to suture soft tissues.

However, in approximately 2 to 3% of all cases, infections occur after surgery or in a surgical site, and as a cause of the infections, contamination of the suture has been identified. For example, during the surgical treatment or surgery, bacteria or germs enter the surgical site from the surroundings to be attached to the suture. Specifically, the bacteria or germs may spread to surrounding tissues by means of the suture, which causes propagation (or colonization) of bacteria or germs to cause the infection or trauma of the patient.

In order to solve this problem, the suture of the related art is manufactured, sterilized, and then packaged in a specialized facility to be distributed as a package or a kit. The package or kit is opened immediately before being used for the surgery. During the surgery, bacteria or germs in the air may enter the opened package and the suture therein.

In order to solve this problem, antimicrobial or antiseptic materials were included in the package or kit, but the suture which absorbs the antimicrobial material had a drawback of a reduced shelf life.

### Disclosure

### Technical Problem

Accordingly, the present disclosure has been made to solve the problem as described above and an object to be achieved by the present disclosure is to provide an antimicrobial suture packaging kit which has an increased shelf life while suppressing infection of the surgical site due to the contamination of the suture.

Another object of the present disclosure is to provide an antimicrobial suture packaging kit in which a suture and an antimicrobial material are separately stored in one kit to implement a suture which absorbs an antimicrobial material only by a simple pressing operation immediately before use while increasing a shelf life.

Meanwhile, technical objects to be achieved in the present disclosure are not limited to the aforementioned technical objects, and other technical objects, which are not mentioned above, will be apparently understood to a person having ordinary skill in the art from the following description.

### Technical Solution

In order to achieve the above-described technical objects, an antimicrobial suture package kit includes a first accommodation unit 140 which has an internal space sealed by a bonded portion 110 formed by welding two synthetic resin sheets; a suture kit 130 accommodated in the first accommodation unit 140; a second accommodation unit 150 which is located to be adjacent to the first accommodation unit 140 and has an internal space sealed by the bonded portion 110; a liquid antimicrobial material 170 accommodated in the second accommodation unit 150; a partition 300 which partially partitions the first accommodation unit 140 and the second accommodation unit 150 so as to ensure a movement path of the antimicrobial material 170; and an antimicrobial capsule 190 which is accommodated in the second accommodation unit 150 and is filled with the antimicrobial material 170 to be swelled by the antimicrobial material 170, and the antimicrobial material 170 is filled in the antimicrobial capsule 190 to be accommodated separately from the suture kit 130 and passes through a movement path formed by the partition 300 when the second accommodation unit 150 is pressed to rupture the antimicrobial capsule 190, and then flows into the suture kit 130, to be absorbed by the suture 120.

Further, an antimicrobial suture package kit includes a first accommodation unit 140 which has an internal space sealed by a bonded portion 110 formed by welding two synthetic resin sheets; a suture kit 130 accommodated in the first accommodation unit 140; a second accommodation unit 150 which is located to be adjacent to the first accommodation unit 140 and has an internal space sealed by the bonded portion 110; a connection unit 160 which partitions the first accommodation unit 140 and the second accommodation unit 150 between the first accommodation unit 140 and the second accommodation unit 150 and has a second width t2 smaller than a first width t1 of the bonded portion 110; a liquid antimicrobial material 170 accommodated in the second accommodation unit 150; and an antimicrobial capsule 190 which is accommodated in the second accommodation unit 150 while being separated from the suture kit 130 through the connection unit 160 and is filled with the antimicrobial material 170 to be swelled by the antimicrobial material 170, and the antimicrobial material 170 is filled in the antimicrobial capsule 190 to be accommodated separately from the suture kit 130 and the connection unit 160 is opened when the second accommodation unit 150 is pressed to rupture the antimicrobial capsule 190 to allow the antimicrobial material to pass through the connection unit 160, and then flow into the suture kit 130, to be absorbed by the suture 120.

Further, the antimicrobial capsule 190 has any one or more outer appearances of a circle, an oval, or a polygon.

Further, an antimicrobial suture package kit includes a first accommodation unit 140 which has an internal space sealed by a bonded portion 110 formed by welding two synthetic resin sheets; a suture kit 130 accommodated in the first accommodation unit 140; a second accommodation unit 150 which is located to be adjacent to the first accommodation unit 140 and has an internal space sealed by the bonded portion 110; and a connection unit 160 which partitions the first accommodation unit 140 and the second accommodation unit 150 between the first accommodation unit 140 and the second accommodation unit 150 and has a second width t2 smaller than a first width t1 of the bonded portion 110, and the antimicrobial material 170 is accommodated in the second accommodation unit 150 while being separated from the suture kit 130 by the connection unit 160 and the connection unit 160 is opened when the second accommodation unit 150 is pressed to increase an internal pressure to allow the antimicrobial material to pass through the connection unit 160, and then flow into the suture kit 130, to be absorbed by the suture 120.

Further, the antimicrobial material 170 may be selected from a group consisting of halogenated hydroxy ether, acyloxy diphenyl ether, triclosan (2,2,4'-trichloro-2'-hydroxydiphenyl ether), chlorhexidine, and a combination thereof.

Further, the antimicrobial suture package kit further includes a Tyvec package including the antimicrobial suture package kit.

Further, any one or more of a tapered portion 200 and a round portion or a partition 300 parallel to the connection unit 160 is further formed on both sides of the connection unit 160 to induce the antimicrobial material 170 to move toward the connection unit 160.

Further, when the bonded portion 110 is formed, in an area of the bonded portion 110 in which the connection unit 160 is formed, a condition in which a bonding strength is weaker than that of welding the bonded portion 110 is used and when the second accommodation unit 150 is pressed, the connection unit 160 having a relatively weak bonding strength is opened to move the antimicrobial material 170 to the first accommodation unit 140.

### Advantageous Effects

According to the exemplary embodiment of the present disclosure, the infection of the surgical site may be suppressed by the antimicrobial material which is absorbed in the suture.

Further, the suture and the antimicrobial material are separately accommodated in one kit to significantly increase the shelf life and conveniently distribute and store the kit.

Further, a suture which absorbs an antimicrobial material may be implemented only by a simple pressing operation immediately before use. Accordingly, it is hygienic and convenient to use and a transparent packaging may allow visual confirmation of movement and absorption of the antimicrobial material.

Effects to be achieved by the present disclosure are not limited to the aforementioned effects, and other effects which have not been mentioned will be obviously understood by those skilled in the art from the description below.

### Description of Drawings

The accompanying drawings of this specification exemplify a preferred embodiment of the present disclosure, the spirit of the present disclosure will be more clearly understood from the following detailed description taken in conjunction with the accompanying drawings, and thus it will be understood that the present disclosure is not limited to only contents illustrated in the accompanying drawings.
FIG. 1 is a planar cross-sectional view and a side view of an antimicrobial suture packaging kit according to a first exemplary embodiment of the present disclosure.
FIG. 2 is a planar cross-sectional view and a side view of a first modified embodiment of an antimicrobial suture packaging kit illustrated in FIG. 1.
FIG. 3 is a planar cross-sectional view and a side view of a second modified embodiment of an antimicrobial suture packaging kit illustrated in FIG. 1.
FIG. 4 is a planar cross-sectional view and a side view of a third modified embodiment of an antimicrobial suture packaging kit illustrated in FIG. 1.
FIG. 5 is a planar cross-sectional view and a side view of an antimicrobial suture packaging kit according to a second exemplary embodiment of the present disclosure.
FIG. 6 is a side view of a modified embodiment of an antimicrobial suture packaging kit illustrated in FIG. 5.
FIG. 7 is an operation status view of FIG. 5.

### Best Mode

Hereinafter, with reference to the attached drawings, an embodiment of the present disclosure is described in detail so that a person having ordinary skill in the art to which the present disclosure pertains can easily carry out the present disclosure. Description of the present disclosure is just an embodiment for structural and functional description so that the scope of the present disclosure is not interpreted to be limited by the embodiment described in the specification. That is, the embodiment may be modified in various forms so that it is understood that the scope of the present disclosure has equivalents which are capable of implementing the technical spirit. Further, it does not mean that the specific embodiment includes the object or effect proposed in the present disclosure or includes only the effect so that it is not understood that the scope of the present disclosure is limited thereby.

In the meantime, meanings of terms described in the present disclosure can be understood as follows.

The terms "first" or "second" are used to distinguish one component from the other component so that the scope should not be limited by these terms. For example, a first component may be referred to as a second component, and similarly, a second component may be referred to as a first component. It should be understood that, when it is described that an element is "connected" to another element, the element may be directly connected to the other element or connected to the other element through a third element. In contrast, it should be understood that, when it is described that an element is "directly connected" to another element, no element is present between the element and the other element. Other expressions which describe the relationship between components, that is, "between" and "directly between", or "adjacent to" and "directly adjacent to" need to be interpreted by the same manner.

Singular expressions should be understood to include plural expressions unless the context apparently indicates otherwise, and it should be understood that terms "include" or "have" indicate that a feature, a number, a step, an operation, a component, a part or the combination thereof described in the specification is present, but do not exclude a possibility of presence or addition of one or more other features, numbers, steps, operations, components, parts or combinations thereof, in advance.

Unless they are contrarily defined, all terms used herein have the same meaning as those generally understood by a person with ordinary skill in the art to which the present disclosure pertains. Terms which are defined in a generally used dictionary should be interpreted to have the same meaning as the meaning in the context of the related art, but are not interpreted as an ideally or excessively formal meaning if it is not clearly defined in the present disclosure.

### Configuration of first exemplary embodiment

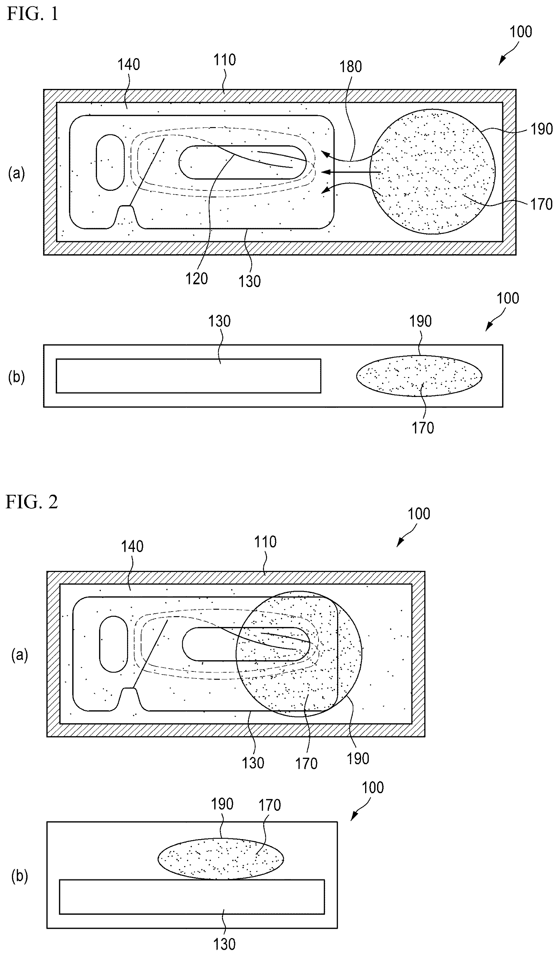

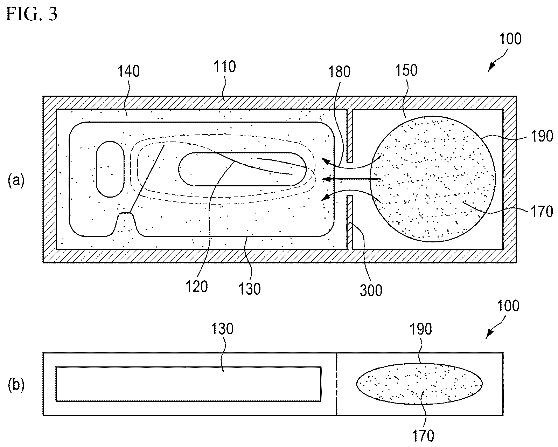

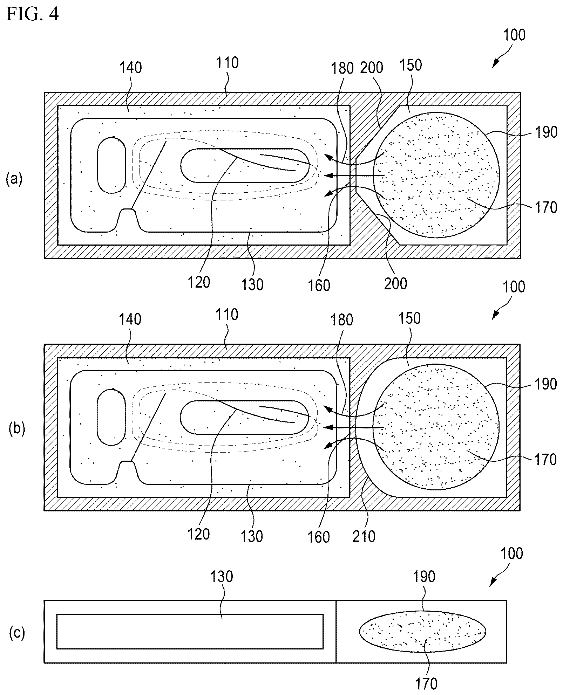

Hereinafter, a configuration of a first exemplary embodiment will be described referring to accompanying drawings. FIG. 1 is a planar cross-sectional view and a side view of an antimicrobial suture packaging kit according to a first exemplary embodiment of the present disclosure, FIG. 2 is a planar cross-sectional view and a side view of a first modified embodiment of an antimicrobial suture packaging kit illustrated in FIG. 1, FIG. 3 is a planar cross-sectional view and a side view of a second modified embodiment of an antimicrobial suture packaging kit illustrated in FIG. 1, and FIG. 4 is a planar cross-sectional view and a side view of a third modified embodiment of an antimicrobial suture packaging kit illustrated in FIG. 1. As illustrated in FIGS. 1 to 4, the antimicrobial suture packaging kit 100 is manufactured by welding two synthetic resin sheets. The synthetic resin sheet is manufactured by a low-density polyethylene (LDPE), PE, PVC, and vinyl material, and two sheets are bonded and then welded.

The antimicrobial suture packaging kit 100 has an approximately rectangular shape and a bonded portion 110 with a first width t1 (for example, 3 to 5 mm) is formed along an edge thereof. The bonded portion 110 partitions and seals a first accommodation unit 140 and a second accommodation unit 150 so as not to communicate with each other.

The first accommodation unit 140 accommodates a previously manufactured suture kit 130 in which the suture 120 is wound and both ends are exposed to be easily withdrawn.

The second accommodation unit 150 is located to be adjacent to the first accommodation unit 140 and an internal space thereof is sealed by the bonded portion 110. A planar area of the second accommodation unit 150 is approximately half that of the first accommodation unit 140. In the second accommodation unit 150, an antimicrobial capsule 190 filled with a liquid antimicrobial material 170 is accommodated.

The antimicrobial material 170 may be selected from a group consisting of halogenated hydroxy ether, acyloxy diphenyl ether, triclosan (2,2,4'-trichloro-2'-hydroxydiphenyl ether), chlorhexidine, and a combination thereof.

A connection unit 160, as illustrated in FIG. 4, may be provided in an antimicrobial suture packaging kit 100 obtained by modifying the antimicrobial suture packaging kit 100 of FIG. 1.

The connection unit 160 partitions the first accommodation unit 140 and the second accommodation unit 150 between the first accommodation unit 140 and the second accommodation unit 150 and in contrast, when the connection unit 160 is opened by a user, the partition of the first accommodation unit 140 and the second accommodation unit 150 is undone.

Such a connection unit 160 has a second width t2 (for example, 1 to 2 mm) smaller than the first width t1 of the bonded portion 110. When the user presses the second accommodation unit 150, the connection unit 160 is opened to allow the antimicrobial material 170 to move toward the first accommodation unit 140.

As another example, when two synthetic resin sheets are welded, a condition providing a weaker bonding strength than that used for welding the bonded portion 110 is used in an area of the bonded portion 110 where the connection unit 160 is to be formed so that when the user presses the second accommodation unit 150 with a finger, the connection unit 160 having a relatively weak bonding strength is opened to allow the antimicrobial material 170 to move to the first accommodation unit 140.

Further, a tapered portion 200 which is narrowed toward the first accommodation unit 140 is further formed on both sides of the connection unit 160 to induce the antimicrobial material 170 to move toward the connection unit 160 in the center.

However, the present disclosure is not limited to forming the tapered portion 200 on both sides of the connection unit 160, and a round portion 210 or a partition 300 having an inner surface different from the tapered portion 200 may also be formed.

The partition 300 is provided in an antimicrobial suture packaging kit 100 obtained by modifying the antimicrobial suture packaging kit 100 of FIG. 1.

Further, the partition 300 is formed on both sides of the connection unit 160 to be parallel to the connection unit 160, instead of the tapered portion 200 illustrated in FIG. 4 or is formed without using the connection unit 160, as illustrated in FIG. 3, to ensure a movement path of the antimicrobial material 170. At this time, the first accommodation unit 140 and the second accommodation unit 150 are partially partitioned.

Due to the characteristic of the shape of the partition 300, as compared with an example that the tapered portion 200 is formed on both sides of the connection unit 160, the space for the second accommodation unit 150 can be ensured and a charging rate of the antimicrobial material 170 can be improved.

The entire antimicrobial suture packaging kit 100 is packed by a Tyvec material to prevent damage or contamination during the distribution. The Tyvec is a product using Tyvek (HDPE, nonwoven fabric) of DuPont in the United States and is mainly used to store and pack sensitive electronic products or medical supplies.

The Tyvec package has an excellent pin-hole property and is highly resistant to oxygen permeation and has a durability so that it is not easily torn even under the external impact. Further, it has a multi-layered structure in which a plurality of PE layers, aluminum layers, or LDPE layers is bonded to completely block external moisture and completely protect the contents.

### Operation of first exemplary embodiment

Hereinafter, an operation of a first exemplary embodiment will be described in detail with reference to FIG. 1. First, the antimicrobial suture package kit 100 is manufactured, distributed and stored in a state as illustrated in FIG. 1.

In the antimicrobial suture package kit 100, an antimicrobial capsule 190 may be disposed on one side and a suture kit 130 may be disposed on the other side. The antimicrobial material 170 is filled in the antimicrobial capsule 190 so as to allow the antimicrobial capsule 190 to swell to be accommodated separately from the suture kit 130.

At this time, the antimicrobial capsule 190 may be any one or more of a circle, an oval, or a polygon and may swell by the filled antimicrobial material 170.

In order to use the antimicrobial suture package kit 100, the user presses the antimicrobial capsule 190 with a finger to rupture and the antimicrobial material 170 with increased internal pressure is discharged from the antimicrobial capsule 190 to move in a movement direction 180 illustrated in FIG. 1 toward the suture kit 130.

While the antimicrobial material 170 moves along the movement direction 180 to pass through the connection unit 160, the antimicrobial material 170 flows into the suture kit 130 to be absorbed in the suture 120.

At this time, an antimicrobial material 170 may be filled in the antimicrobial capsule 190 in an amount sufficient to be absorbed by the entire suture 120.

Further, the user may optionally hold the antimicrobial suture package kit 100 in an upright position so as to shorten a time for the antimicrobial material 170 to flow into the suture kit 130.

In the meantime, the user may visually confirm the process of moving the antimicrobial material 170 to be absorbed by the suture 120 and when the user determines that an absorption process is sufficient, the user cuts the bonded portion 110, takes out the suture kit 130 to perform the surgery using the suture 120.

### Operation of first modified embodiment

Hereinafter, an operation of a first modified embodiment will be described in detail with reference to FIG. 2. First, the antimicrobial suture package kit 100 is manufactured, distributed, and stored in a state as illustrated in FIG. 2.

In the antimicrobial suture package kit 100, an antimicrobial capsule 190 and the suture kit 130 may be disposed so as to overlap in at least one direction of up/down/left/right. The antimicrobial material 170 is filled in the antimicrobial capsule 190 so as to allow the antimicrobial capsule 190 to swell to be accommodated separately from the suture kit 130.

The antimicrobial capsule 190 of the first modified embodiment may be implemented to be the same as that described in the operation of the first exemplary embodiment.

In order to use the antimicrobial suture package kit 100, the user presses the antimicrobial capsule 190 with a finger to rupture so that the antimicrobial material 170 with an increased internal pressure is discharged from the antimicrobial capsule 190.

Thereafter, the antimicrobial material 170 flows into the suture kit 130 to be absorbed by the suture 120 and subsequent operation and use are the same as those described in the operation of the first exemplary embodiment.

### Operation of second modified embodiment

Hereinafter, an operation of a second exemplary embodiment will be described in detail with reference to FIG. 3. First, the antimicrobial suture package kit 100 is manufactured, distributed, and stored in a state as illustrated in FIG. 3.

In the antimicrobial suture package kit 100, the antimicrobial capsule 190 is accommodated in the second accommodation unit 150 and the first accommodation unit 140 and the second accommodation unit 150 may be partially partitioned by the partition 300. Further, the antimicrobial material 170 is filled in the antimicrobial capsule 190 to swell the antimicrobial capsule 190 to be accommodated separately from the suture kit 130.

The antimicrobial capsule 190 of the second modified embodiment may be implemented to be the same as that described in the operation of the first exemplary embodiment.

In order to use the antimicrobial suture package kit 100, the user presses the antimicrobial capsule 190 with a finger to rupture and the antimicrobial material 170 with increased internal pressure is discharged from the antimicrobial capsule 190 to move in a movement direction 180 illustrated in FIG. 3 toward the suture kit 130. The antimicrobial material 170 may pass through the movement path formed by the partition 300 while moving to the movement direction 180.

Thereafter, the antimicrobial material 170 flows into the suture kit 130 to be absorbed by the suture 120 and subsequent operation and use are the same as those described in the operation of the first exemplary embodiment.

### Operation of third modified embodiment

Hereinafter, an operation of a third modified embodiment will be described in detail with reference to FIG. 4. First, the antimicrobial suture package kit 100 is manufactured, distributed, and stored in a state as illustrated in FIG. 4.

In the antimicrobial suture package kit 100, the antimicrobial capsule 190 may be accommodated in the second accommodation unit 150 to be separated from the suture kit 130 by the connection unit 160 and the antimicrobial material 170 may be filled in the antimicrobial capsule 190 to swell the antimicrobial capsule 190.

The antimicrobial capsule 190 of the third modified embodiment may be implemented to be the same as that described in the operation of the first exemplary embodiment.

In order to use the antimicrobial suture package kit 100, the user presses the antimicrobial capsule 190 with a finger to rupture, thereby discharging the antimicrobial material 170 with an increased internal pressure from the antimicrobial capsule 190 to be moved to the movement direction 180 illustrated in FIG. 4. The connection unit 160 may be opened as the antimicrobial material 170 is discharged from the antimicrobial capsule 190, thereby undoing the partition of the first accommodation unit 140 and the second accommodation unit 150.

Thereafter, the antimicrobial material 170 flows into the suture kit 130 to be absorbed by the suture 120 and subsequent operation and use are the same as those described in the operation of the first exemplary embodiment.

### Configuration of second exemplary embodiment

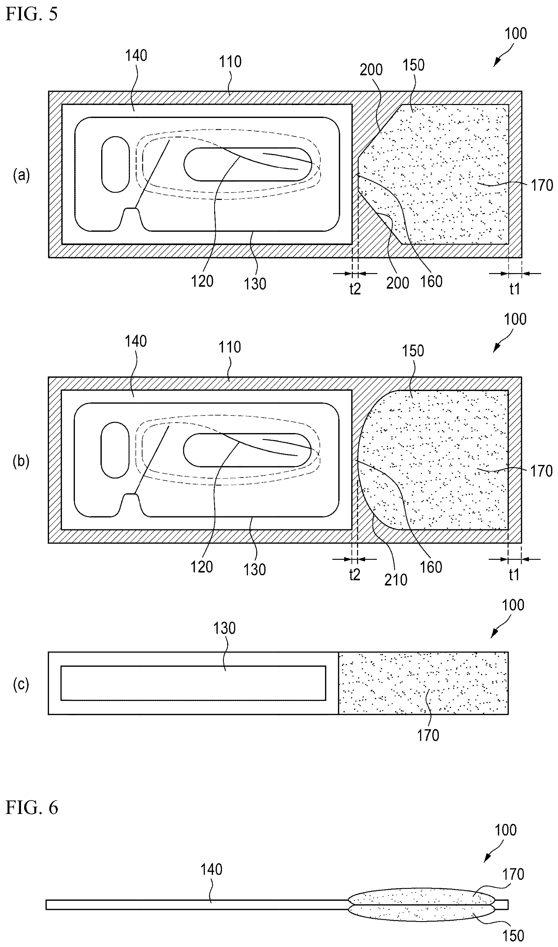

Hereinafter, with reference to the accompanying drawings, for the convenience of the description, the common feature with the first exemplary embodiment will be omitted and the configuration of the second exemplary embodiment will be described in detail with a focus on the difference from the first exemplary embodiment. FIG. 5 is a planar cross-sectional view and a side view of an antimicrobial suture packaging kit according to a second exemplary embodiment of the present disclosure. FIG. 6 is a side view of a modified embodiment of an antimicrobial suture packaging kit illustrated in FIG. 5.

Referring to FIG. 5, in an antimicrobial suture package kit 100, not an antimicrobial capsule 190, but an antimicrobial material 170 may be accommodated in the second accommodation unit 150 while being separated from the suture kit 130 through the connection unit 160.

In the second exemplary embodiment, the second accommodation unit 150 is located to be adjacent to the first accommodation unit 140 and an internal space is sealed by the bonded portion 110. A planar area of the second accommodation unit 150 is approximately half that of the first accommodation unit 140. In the second accommodation unit 150, a liquid antimicrobial material 170 is filled to be accommodated. Therefore, as illustrated in FIG. 2, the second accommodation unit 150 is in a swelling state. As illustrated in FIG. 2, a thickness of the second accommodation unit 150 filled with the antimicrobial material 170 is two to three times larger than the thickness of the first accommodation unit 140.

Further, also in the first exemplary embodiment, the second accommodation unit 150 may be provided on the antimicrobial suture package kit 100 to be two or three times thicker than the thickness of the first accommodation unit 140 as illustrated in FIG. 6.

### Operation of second exemplary embodiment

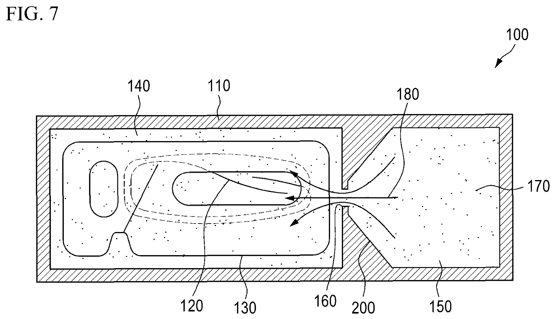

Hereinafter, an operation of a second exemplary embodiment will be described in detail with reference to FIG. 7. FIG. 7 is an operation state view of FIG. 5 and the antimicrobial suture package kit 100 is manufactured, distributed, and stored in a state as illustrated in FIG. 5.

In order to use the antimicrobial suture package kit 100, the user presses the second accommodation unit 150 with a finger immediately before the surgery to increase an internal pressure of the antimicrobial material 170 and open the connection unit 160 to move the antimicrobial material 170 with an increased internal pressure to a movement direction 180 illustrated in FIG. 7.

While the antimicrobial material 170 moves along the movement direction 180 to pass through the connection unit 160, the antimicrobial material 170 flows into the suture kit 130 to be absorbed by the suture 120.

At this time, an antimicrobial material 170 may be filled in the second accommodation unit 150 in an amount sufficient to be absorbed by the entire suture 120.

Further, the user may optionally hold the antimicrobial suture package kit 100 in an upright position so as to shorten a time for the antimicrobial material 170 to flow into the suture kit 130.

In the meantime, the user may visually confirm the process of moving the antimicrobial material 170 to be absorbed by the suture 120 and when the user determines that an absorption process is sufficient, the user cuts the bonded portion 110, takes out the suture kit 130 to perform the surgery using the suture 120.

### First experimental example

In a first experimental example, in order to conduct the test for the bonding strength of the bonded portion 110 formed by welding two synthetic resin sheets in accordance with the international standard ASTM F88/F88M-21, a pouch film which replaces the bonded portion 110 may be thermally welded at intervals of 10 mm × 15 mm at a temperature range of 0 to 200°C.

Thereafter, the pouch film is cut in a predetermined size with the thermally welded portion at the center and a head speed of a universal testing machine for the bonding strength test may be set to 200 mm/min.

Thereafter, the bonding strength was measured by attaching a jig to the universal testing machine, fixing the pouch film to the jig with the bonded surface at the center, and pulling upward at a constant speed. A measurement result of the bonding strength of the pouch film is represented in the following Table 1.

**[Table 1]**

| | Measurement range | Measured value |
|---|---|---|
| Bonding strength | Upper limit | 100 N |
| | Lower limit | 1 N |

### Second experimental example

In a second experimental example, in order to conduct the test for a compressive force of the antimicrobial capsule 190, an antimicrobial capsule 190 filled with distilled water, instead of the antimicrobial material 170, may be manufactured in a predetermined size (for example, approximately 20 to 30 mm).

Thereafter, the compressive force was measured by attaching a jig to the universal testing machine, placing the antimicrobial capsule 190 in the middle of the jig, and pressing the antimicrobial capsule 190 at a constant speed to measure a compressive force at which the antimicrobial capsule 190 is ruptured and the measurement result of the compressive force of the antimicrobial capsule 190 is represented in the following Table 2.

**[Table 2]**

| | Measurement range | Measurement value |
|---|---|---|
| Compressive force | Upper limit | 100 N |
| | Lower limit | 0.1 N |

In Table 2, the upper limit refers to a maximum compressive force of the antimicrobial capsule 190 at which the antimicrobial capsule is ruptured by an adult and the lower limit refers to a minimum compressive force of the antimicrobial capsule 190 at which an outer appearance (shape) is maintained during transport, dropping, vibration, or storage.

As described above, the detailed description of the preferred exemplary embodiments of the disclosed present disclosure is provided such that those skilled in the art implement and carry out the present disclosure. While the disclosure has been described with reference to the preferred embodiments, it will be understood by those skilled in the art that various changes and modifications of the present disclosure may be made without departing from the spirit and scope of the disclosure. For example, those skilled in the art may use configurations disclosed in the above-described exemplary embodiments by combining them with each other. Therefore, the present disclosure is not intended to be limited to the above-described exemplary embodiments but to assign the widest scope consistent with disclosed principles and novel features.

The present disclosure may be implemented in another specific form within the scope without departing from the spirit and essential feature of the present disclosure. Therefore, the detailed description should not restrictively be analyzed in all aspects and should be exemplarily considered. The scope of the present disclosure should be determined by rational interpretation of the appended claims and all changes are included in the scope of the present disclosure within the equivalent scope of the present disclosure. The present disclosure is not intended to be limited to the above-described exemplary embodiments but to assign the widest scope consistent with disclosed principles and novel features. Further, claims having no clear quoting relation in the claims are combined to configure the embodiment or may be included as new claims by correction after application.

### Industrial Applicability

The antimicrobial suture package kit of the present disclosure has advantages of preventing infection of a surgical site by the antimicrobial material absorbed in the suture, separately accommodating the suture and the antimicrobial material in one kit to significantly extend the shelf life, easily distributing and storing the kit, implementing the suture in which the antimicrobial material is absorbed only by a simple pressing operation immediately before use, and being hygienic, easy to use, and allowing visual confirmation of movement and absorption of the antiracial material by the transparent package, thereby making it industrially applicable.

## Claims

1. An antimicrobial suture package kit, comprising:
a first accommodation unit (140) which has an internal space sealed by a bonded portion (110) formed by welding two synthetic resin sheets;
a suture kit (130) accommodated in the first accommodation unit (140);
a second accommodation unit (150) which is located to be adjacent to the first accommodation unit (140) and has an internal space sealed by the bonded portion (110);
a liquid antimicrobial material (170) accommodated in the second accommodation unit (150);
a partition (300) which partially partitions the first accommodation unit (140) and the second accommodation unit (150) so as to ensure a movement path of the antimicrobial material (170); and
an antimicrobial capsule (190) which is accommodated in the second accommodation unit (150) and is filled with the antimicrobial material (170) to be swelled by the antimicrobial material (170),
wherein the antimicrobial material (170) is filled in the antimicrobial capsule (190) to be accommodated separately from the suture kit (130) and passes through the movement path formed by the partition 300 when the second accommodation unit (150) is pressed to rupture the antimicrobial capsule (190), and then flows into the suture kit (130), to be absorbed by the suture (120).

2. An antimicrobial suture package kit, comprising:
a first accommodation unit (140) which has an internal space sealed by a bonded portion (110) formed by welding two synthetic resin sheets;
a suture kit (130) accommodated in the first accommodation unit (140);
a second accommodation unit (150) which is located to be adjacent to the first accommodation unit (140) and has an internal space sealed by the bonded portion (110);
a connection unit (160) which partitions the first accommodation unit (140) and the second accommodation unit (150) between the first accommodation unit (140) and the second accommodation unit (150) and has a second width (t2) smaller than a first width (t1) of the bonded portion (110);
a liquid antimicrobial material (170) accommodated in the second accommodation unit (150); and
an antimicrobial capsule (190) which is accommodated in the second accommodation unit (150) while being separated from the suture kit (130) through the connection unit (160) and is filled with the antimicrobial material (170) to be swelled by the antimicrobial material (170),
wherein the antimicrobial material (170) is filled in the antimicrobial capsule (190) to be accommodated separately from the suture kit (130) and the connection unit (160) is opened when the second accommodation unit (150) is pressed to rupture the antimicrobial capsule (190) to allow the antimicrobial material to pass through the connection unit (160), and then flow into the suture kit (130), to be absorbed by the suture (120).

3. The antimicrobial suture package kit of claim 1 or 2, wherein the antimicrobial capsule (190) has an outer shape of any one or more of a circle, an oval, or a polygon.

4. An antimicrobial suture package kit, comprising:
a first accommodation unit (140) which has an internal space sealed by a bonded portion (110) formed by welding two synthetic resin sheets;
a suture kit (130) accommodated in the first accommodation unit (140);
a second accommodation unit (150) which is located to be adjacent to the first accommodation unit (140) and has an internal space sealed by the bonded portion (110); and
a connection unit (160) which partitions the first accommodation unit (140) and the second accommodation unit (150) between the first accommodation unit (140) and the second accommodation unit (150) and has a second width (t2) smaller than a first width (t1) of the bonded portion (110),
wherein the antimicrobial material (170) is accommodated in the second accommodation unit (150) while being separated from the suture kit (130) by the connection unit (160) and the connection unit (160) is opened when the second accommodation unit (150) is pressed to increase an internal pressure to allow the antimicrobial material to pass through the connection unit (160), and then flow into the suture kit (130), to be absorbed by the suture (120).

5. The antimicrobial suture package kit of any one of claims 1, 2, and 4, wherein the antimicrobial material (170) is selected from a group consisting of halogenated hydroxy ether, acyloxy diphenyl ether, triclosan (2,2,4'-trichloro-2'-hydroxydiphenyl ether), chlorhexidine, and a combination thereof.

6. The antimicrobial suture package kit of any one of claims 1, 2, and 4, further comprising:
a Tyvec package including the antimicrobial suture package kit.

7. The antimicrobial suture package kit of claim 2 or 4, wherein any one or more of a tapered portion (200) and a round portion (210) or a partition (300) parallel to the connection unit (160) is further formed on both sides of the connection unit (160) to induce the antimicrobial material (170) to move toward the connection unit (160).

8. The antimicrobial suture package kit of claim 2 or 4, wherein when the bonded portion (110) is formed, in an area of the bonded portion (110) in which the connection unit (160) is formed, a condition in which a bonding strength is weaker than that of welding the bonded portion (110) is used and
when the second accommodation unit (150) is pressed, the connection unit (160) having a relatively weak bonding strength is opened to allow the antimicrobial material (170) to move toward the first accommodation unit (140).
